**Europäisches Patentamt**

**European Patent Office**

**Office européen des brevets**

(11) Veröffentlichungsnummer: **0 451 591 A1**

## (12) EUROPÄISCHE PATENTANMELDUNG

(21) Anmeldenummer: **91104651.4**

(51) Int. Cl.5: **C12Q 1/68**, C12N 15/10

(22) Anmeldetag: **25.03.91**

(30) Priorität: **31.03.90 DE 4010465**

(43) Veröffentlichungstag der Anmeldung:
**16.10.91 Patentblatt 91/42**

(84) Benannte Vertragsstaaten:
**AT BE CH DE DK ES FR GB GR IT LI LU NL SE**

(71) Anmelder: **BOEHRINGER MANNHEIM GMBH**
**Sandhofer Strasse 116**
**W-6800 Mannheim 31(DE)**

(72) Erfinder: **Kessler, Christoph, Dr.rer.nat.**
**Schlossbergweg 11**
**W-8021 Dorfen(DE)**
Erfinder: **Rüger, Rüdiger, Dr.med.**
**Tutzinger Strasse 2**
**W-8124 Seeshaupt(DE)**

(54) **Verfahren zur spezifischen Vervielfältigung von Nukleinsäure-templates und zur Bestimmung von Nukleinsäuren.**

(57) Ein Verfahren zur spezifischen Vervielfältigung und zum Nachweis von Nukleinsäure-templates durch Hybridisierung von zwei Nukleinsäure-primern an dieses template, Bildung einer template-komplementären Nukleinsäure mit diesen primern und Transkription der gebildeten Nukleinsäure zu einer Mehrzahl von template-analogen Nukleinsäuren, dadurch gekennzeichnet, daß das template mit zwei primern, die in gleicher Orientierung angeordnet sind, komplementäre Sequenzen zum template enthalten und von denen der zweite primer an dem dem ersten zweiten primer abgewandten Ende eine Transkriptionsinitiationsstelle und eine doppelsträngige Sequenz, an die eine RNA-Polymerase binden kann, trägt, hybridisiert wird, die beiden so hybridisierten primer durch Auffüllen der Lücke zwischen ihnen zu einer template-komplementären Nukleinsäure kovalent verknüpft werden, Transkripte von dieser template-komlementären Nukleinsäure gebildet werden und diese Transkripte gegebeneenfalls durch an sich bekannte Weise nachgewiesen werden.

EP 0 451 591 A1

Gegenstand der Erfindung ist ein Verfahren zur spezifischen Vervielfältigung von Nukleinsäure-templates und ein Verfahren zur Bestimmung von Nukleinsäuren.

Verfahren zur spezifischen Vervielfältigung von Nukleinsäure-templates sind beispielsweise in der EP-A 0 200 362 beschrieben. Dort wird vorgeschlagen, die nachzuweisende Nukleinsäure durch ein in vitro-System zu vermehren. Dazu wird der Probe für jeden zu vervielfältigenden Nukleinsäure-Einzelstrang mindestens ein primer zugesetzt. Durch eine enzymatische Verlängerungsreaktion wird zu jedem der Nukleinsäure-Einzelstränge, ausgehend von dem primer, ein komplementärer Nukleinsäurestrang gebildet. Diese Reaktion kann mehrmals hintereinander durchgeführt werden, wobei auch die neugebildeten Nukleinsäurestränge vervielfältigt werden können. Nachteil dieses Verfahrens ist, daß zwischen jedem Vervielfältigungsschritt ein Erhitzungsschritt erforderlich ist.

In der EP-A 0 329 822 wird ein Verfahren zur Vervielfältigung von spezifischen Nukleinsäure-templates durch Hybridisierung von zwei Nukleinsäure-primern an dieses template, templateabhängige Bildung von DNA mit diesen primern und Transkription dieser DNA zu einer Mehrzahl von template-analogen Nukleinsäuren beschrieben. Der Nachteil dieses Verfahrens liegt insbesondere darin, daß das Verfahren lange Inkubationszeiten bei relativ hohen Temperaturen erfordert. Außerdem ist das Verfahren zur Vervielfältigung von DNA nur nach aufwendiger Vorbehandlung des templates geeignet.

Aufgabe der vorliegenden Erfindung war es, die geschilderten Nachteile des Standes der Technik zu beseitigen und ein einfacheres, besonders empfindliches Verfahren zur Vervielfältigung und zum spezifischen Nachweis von Nukleinsäure-templates bereitzustellen.

Gegenstand der Erfindung ist ein Verfahren zur spezifischen Vervielfältigung von Nukleinsäure-templates durch Hybridisierung von zwei Nukleinsäure-primern an dieses template, Bildung einer template-komplementären Nukleinsäure mit diesen primern und Transkription der gebildeten Nukleinsäure zu einer Mehrzahl von template-analogen Nukleinsäuren, dadurch gekennzeichnet,

daß das template mit zwei primern, die in gleicher Orientierung angeordnet sind, komplementäre Sequenzen zum template enthalten und von denen der zweite primer an dem dem ersten primer abgewandten Ende eine Transkriptionsinitiationsstelle und eine doppelsträngige Sequenz, an die eine RNA- Polymerase binden kann, trägt, hybridisiert wird,

die beiden so hybridisierten primer durch Auffüllen der Lücke zwischen ihnen zu einer template-komplementären Nukleinsäure kovalent verknüpft werden und Transkripte (template-analoge Nucleinsäuren) von dieser template-komplementären Nukleinsäure gebildet werden.

In einer bevorzugten Ausführungsform werden die gebildeten Transkripte erneut als templates zur Hybridisierung mit den primern verwendet und der Vervielfältigungszyklus wiederholt. In einer weiter bevorzugten Ausführungsform können insbesondere zur Erhöhung der Spezifität weitere primer verwendet werden, die sich im template-komplementären Bereich von dem im ersten Zyklus verwendeten primer unterscheiden.

Unter Nukleinsäure-templates im Sinne der Erfindung ist DNA und RNA prokaryontischer oder eukaryontischer Herkunft zu verstehen. Dazu gehören auch virale und bakterielle Nukleinsäuren sowie Nukleinsäuren von Viroiden. Sie können einzel- oder doppelsträngig sein. Es kann sich um episomale Nukleinsäuren, wie Plasmide, oder genomische chromosomale Nukleinsäuren handeln. Die Nukleinsäuren können für einen bestimmten Organismus oder eine Gruppe von Organismen charakteristisch sein.

Die Nukleinsäuren können auch als Rohlysat oder gereinigt (z.B. durch Phenolextraktion oder Guanidin/Isothiocyanat-Gratientenzentrefugation) verwendet werden.

Es können aber auch modifizierte Nukleinsäuren verwendet werden, beispielsweise mittels Restriktionsenzymen geschnittene Nukleinsäuren oder um durch Exonucleasenbehandlung modifizierte Nucleinsäuren. Im Fall von RNA kann vorher cDNA hergestellt werden. Als vorteilhaft hat es sich erwiesen, wenn die Nukleinsäuren vor der Durchführung der Reaktion in einzelsträngiger Form vorliegen oder in einzelsträngige Form gebracht werden.

Unter einem primer ist eine Nukleotidsequenz zu verstehen, welche zum Nukleinsäure-template komplementäre Sequenzen enthält und dadurch mit dem template unter stringenten Bedingungen hybridisieren kann. (z.B. Anal. Biochem. 138 (1984) 267-284).

Unter einer template-komplementären Nucleinsäure ist eine Nukleinsäure zu verstehen, die mindestens in einem Teilbereich komplementär zum template ist. Unter einer template - analogen Nukleinsäure ist eine Nukleinsäure zu verstehen, die über das Vervielfältigungsverfahren hergestellt wurde und dadurch in mindestens einem Teilbereich den zu den Primern komplementären Sequenzen und der aufgefüllten Lückensequenz entspricht, in der die Sequenz homolog zu der template Sequenz ist.

Als zweiter primer wird ein Nukleinsäure-Fragment verwendet, welches eine komplementäre Sequenz zum template mit vorzugsweise 15 - 40, besonders bevorzugt 16 - 25 Basen enthält. An diese, die komplementäre Sequenz enthaltende Einzelstrandsequenz, schließt sich eine Transkriptionsinitiationsstelle und eine doppelsträngige Sequenz, an die eine RNA-Polymerase binden kann, an. Die doppelsträngige Sequenz hat vorzugsweise eine Länge von 17 - 100 Basen, besonders bevorzugt 7 -50 Basen. Die beiden Stränge des doppelsträngigen Anteils können entweder offen vorliegen oder an den beiden Enden, die der template-komplementären Sequenz abgewandt sind, über eine weitere Nukleinsäure-Sequenz welches vorzugsweise eine Länge von 5 - 100, besonders bevorzugt 5 - 10 Basen, Basen hat und vorzugsweise ein Poly-Nukleotid darstellt, verbunden sein.

In einer bevorzugten Ausführungsform beginnt der template-komplementäre Sequenzabschnitt des zweiten primers an dem dem ersten primer zugewandten Ende mit einem phosphorylierten 5'-Ende. Ebenso bevorzugt ist es, daß das dem ersten primer zugewandte 3'-Ende durch ein Didesoxynukleotid, welches besonders bevorzugt komplementär zur Transkriptionsinitiationsstelle oder zum letzten (3') Nucleotid des Promotorabschnitts ist, abschließt.

Geeignete doppelsträngige Sequenzen, an die eine RNA-Polymerase binden kann, sind beispielsweise in Melton et.al NAR 12 (1984) 7035-7056 Pfeiffer, and Gilbert W., Protein Sequences and DNA Analysis 1 (1988) 269-280 beschrieben.

Der erste Primer enthält zum template komplementäre Sequenzen, mit denen eine Hybridisierung des primers unter stringenten Bedingungen (s.o.) an das template ermöglicht wird. Bevorzugt sind solche Bedingungen, unter denen der primer nur an primerspezifische Sequenzen in der template Nukleinsäure bindet.

Die Länge der komplementären Bereiche der primer beträgt vorzugsweise 15 - 40, insbesondere 16 - 25 Basen.

In einer weiteren bevorzugten Ausführungsform enthält einer der beiden primer, an dem dem anderen primer abgewandten Ende kovalent gebunden den Partner eines biologischen Bindungspaares. Darüber hinaus oder alternativ kann die Transkription der template-komplementären Nukleinsäuren zu den template-analogen Nukleinsäuren unter Verwendung von Mononukleotiden, die den Partner eines biologischen Bindungspaares gebunden enthält, anstelle von den unmodifizierten Mononukleotiden vorgenommen werden. Über den an einen Träger immobilisierten anderen Bindungspartner können dann die gebildeten template-komplementären Nukleinsäuren oder/und die Transkripte aus der Reaktionslösung entfernt und immobilisiert werden. Die so immobilisierten Nukleinsäuren können dann beispielsweise durch dem Fachmann geläufige Verfahren nachgewiesen werden.

Beispiele für geeignete Bindungspaare sind Biotin-, Streptavidin- bzw. Avidin, Hapten-Antikörper, Antigen-Antikörper, Konkavalin-Antikörper, Zucker-Lectin oder komplementäre Nukleinsäuren. Bevorzugt werden komplementäre Nukleinsäuren mit der Länge von 5 bis 100, vorzugsweise 10 - 30 Basen verwendet.

In einer bevorzugten Ausführungsform wird das Verfahren zur Vervielfältigung von spezifischen DNA-templates und spezifischen RNA-templates eingesetzt. Falls das DNA-template im Doppelstrang vorliegt, wird das template vor Hybridisierung vorzugsweise in die Einzelstrangform durch dem Fachmann bekannte Verfahren übergeführt.

In einer bevorzugten Ausführungsform wird die zu vervielfältigende oder zu bestimmende Nukleinsäure vor der Hybridisierung mit den primern durch Behandlung mit Restriktionsendonukleasen oder Exonucleasen in ein modifiziertes template übergeführt.

In einer weiteren bevorzugten Ausführungsform wird vor Herstellung der Transkripte die Hybridisierung zwischen template und template-komplementärer DNA aufgehoben. Vorzugsweise erfolgt dies mit einer RNaseH. Besonders bevorzugt wird RNaseH aus E.coli oder aus Kalbsthymus verwendet. Die Konzentration der RNaseH beträgt vorzugsweise 0,5 - 2 U/Reaktionsvolumen.

Die Durchführung der Vervielfältigung erfolgt vorzugsweise bei 30 - 37°C über 30 min bis 2 Stunden. Als Reaktionsvolumen werden vorzugsweise 25 - 100 $\mu$l gewählt. Die Konzentration der primer beträgt vorzugsweise jeweils 0,3 - 3,5 $\mu$mol/l.

Zur Auffüllung der Lücke zwischen den primern wird vorzugsweise eine Ligase und reverse Transkriptase verwendet. Als Ligase wird besonders bevorzugt T4-Ligase verwendet. Die Konzentration der Ligase ist vorzugsweise zwischen 1 und 10 U/Reaktionsvolumen. Ebenso kann die Lücke durch Zugabe eines geeigneten Oligonucleotids und Verknüpfung mit Ligase geschlossen werden. Bei Zugabe von Ligasen muß ein Cofaktor, beispielsweise ATP für $T_4$- oder $NAD^+$ für E.coli Ligase, zugesetzt werden.

Die Konzentration der reversen Transkriptase beträgt vorzugsweise 0 -30 U/Testvolumen. Als reverse Transkriptase wird bevorzugt MoMLV oder AMV reverse Transkriptase verwendet.

Die Herstellung der Transkripte erfolgt durch Zugabe von RNA-Polymerase. Vorzugsweise wird eine phagencodierte RNA-Polymerase, wie beispielsweise T7-RNA-Polymerase, T3-RNA-Polymerase oder SP6-RNA-Polymerase verwendet. Die Konzentration der Polymerase beträgt vorzugsweise 10 - 100 U/Reaktionsvolumen.

Ein weiterer Gegenstand der Erfindung ist ein Verfahren zum Nachweis von Nukleinsäure-templates durch Hybridisierung von zwei Nukleinsäureprimern an dieses template, Bildung einer template-komplementären Nukleinsäure mit diesen primern und Transkription der gebildeten Nukleinsäure zu einer Mehrzahl von template-analogen Nukleinsäuren, dadurch gekennzeichnet,

daß das template mit zwei primern, die in gleicher Orientierung angeordnet sind, komplementäre Sequenzen zum template enthalten und von denen der zweite primer an dem dem erste primer abgewandten Ende eine Transkriptionsinitiationsstelle und eine doppelsträngige Sequenz, an die eine RNA-Polymerase binden kann, trägt, hybridisiert wird,

die beiden so hybridisierten primer durch Auffüllen der Lücke zwischen ihnen zu einer template-komplementären Nukleinsäure kovalent verknüpft werden

Transkripte von dieser template-komplementären Nukleinsäure gebildet werden und diese Transkripte durch in an sich bekannte Weise nachgewiesen werden (z.B. Molecular Cloning 1982, Eds. Maniatis et al., S. 199-206).

Die bevorzugten Ausführungsformen des Nachweisverfahrens sind denen des Vervielfältigungsverfahrens analog.

Die weitere Aufreinigung bzw. der Nachweis der entstandenen Transkriptionsprodukte kann, wie oben beschrieben, über eine Immobilisierung und nachfolgenden Nachweis mit geeigneten Methoden erfolgen. Ebenso geeignet ist eine Auftrennung der Transkriptionsprodukte durch Gel-Elektrophorese, RNA-Anfärbung, Visualisierung, entweder direkt oder durch Northern-Transfer und anschließende Hybridisierung mit markierten target-Sequenz-spezifischen probes. Ebenso geeignet sind dot-,blot/slot-blot-Hybridisierungsverfahren mit markierten target-Sequenz-spezifischen probes sowie Markierung der Transkriptionsprodukte mit einer oder mehreren, beispielsweise radioaktiv-, fluoreszenz- oder enzymmarkierten NTPs.

Durch den Einbau von $^{32}$P-markierten bzw. nicht radioaktiv markierten NTPs können die Produkte im DOT- SLOT- oder Northern-blot direkt sichtbar gemacht werden. Der Einbau von Digoxigenin bzw. Biotin (vgl. WO 89/06698) kann zum direkten Nachweis mit einem biospezifischen Bindepartner, z.B. einem Antidigoxigenin - Antikörper, verwendet werden.

Fig. 1 zeigt die Nukleotidsequenz des in Beispiel 1 eingesetzten ersten Primers.

Fig. 2 zeigt zwei Varianten für den zweiten primer.

Fig. 3 zeigt eine bevorzugte Variante eines Nachweisverfahrens für Nukleinsäuren gemäß der Erfindung.

Die nachfolgenden Beispiele und die Abbildungen erläutern die Erfindung weiter:

Beispiel 1

Herstellung von RNA-templates

Das Plasmid pSPT18 (Sequenz vgl. WO 89/06698) wird zur Produktion von Transkripten des Neomycin-Resistenzgens (neo) verwendet. In dieses Plasmid wird das Neomycin-Gen (eine Aminoglycosid-3'-phosphotransferase II) wie in Beck et al, Gene 19 (1982) 327 - 336, beschrieben, insertiert. Mit dem resultierenden Plasmid pSPT18neo können mit SP6 RNA-Polymerase Transkripte des Gens in der Sense-Orientierung produziert werden. Plasmid pSPT18neo wird mit BglI linearisiert. Aus diesem linearisierten Plasmid werden durch in vitro Transkription, wie in Biochemicals for Molecular Biology, Boehringer Mannheim (1987) Seite 38 - 40 beschrieben, RNA-Transkripte hergestellt, die in Beispiel 2 als templates eingesetzt werden.

Beispiel 2

RNA-Amplifikation

a) Herstellung der primer 1 und 2

Die Sequenz für den primer 1 (DNA-Oligonukleotid von 24 Nukleotiden, Fig. 1) ist komplementär zu einer Region der mRNA des neo-Gens, welche nach Beck et al. den Nukleotiden 2008 - 2031 der DNA-Sequenz entsprechen. Die Sequenz von primer 2 (ebenfalls 24 Nucleotide, Fig. 2) entspricht den Nucleotidpositionen 1937 - 1960 des neo-Gens. Zusätzlich enthält primer 2 die minimal notwendige doppelsträngige Sequenz des Promotors für die RNA-Polymerase des Bakteriophagen T7 (T7 RNAP, Sequenz vgl. Fig. 2) (Uhlenbeck et al., Nature, 328 (1987) 596 - 600). Außerdem enthält primer 2 eine den partiellen Doppelstrang stabilisierende AT-reiche Loop-Region. Zwei funktionelle Varianten der primer-Sequenz sind angegeben in Fig. 2 (ddA bzw. ddG an den 3'-Enden). Primer 2 wird am 5'-Ende phosphoryliert, wie beispielsweise in Maxam and Gilbert in Methods in Enzymology Band 60 (1980) S. 499 and Nucleic Acids Research 3 (1976) 863 beschrieben. Weiter sind, wie ebenfalls dort beschrieben, am 3'-Ende ddA bzw. ddC angefügt.

b) Amplifikationsreaktion

Reaktionsgemisch:
40 mmol/l Tris HCl (pH 8 bei 37°C),
10 mmol/l, DTT,
1 mmol/l Spermidin,
0,01 % Triton X 100,
8 % Polyethylenglycol,
20 mmol/l MgCl$_2$,
1 mmol/l ATP,

je 2 mmol/l NTPs,
je 1 mmol/l dNTPs,
500 nmol/l primer 1,
1 µmol/l primer 2,
5 U/Reaktionsvolumen T4 Ligase,
40 U/Reaktionsvolumen T7 RNAP,
20 U/Reaktionsvolumen reverse Transkriptase und
1 U/Reaktionsvlolumen RNase H.

Die nicht enzymatischen Einsatzstoffe werden vor Verwendung mit 0,1 % Dietylpyrocarbonat analog Maniatis (s.u.) Seiten 7.3 - 7.4 vorbehandelt.

In ein Reaktionsgefäß von 50 µl Inhalt wird die Probe (RNA-Fragment nach Beispiel 1 bzw. Verdünnungen davon) zugegeben und mit dem Reaktionsgemisch aufgefüllt.

Der Ansatz wird zwei Stunden bei 37° C inkubiert, die produzierte RNA mit Ethanol ausgefällt, in einem RNA-Gel aufgetrennt, gefärbt und auf eine Nylonmembran, wie in Molecular Cloning, 1989, Editiors Sambrook et al., CSH, Seiten 7.43 - 7.51 beschrieben,
transferiert. Die Membran-gebundene RNA kann mit neospezifischen Proben, die Digoxigenin-markiert sind, (Herstellung nach "DNA-Labeling and Detection", Boehringer Mannheim GmbH, 1989, Seite 27 - 28 oder nach WO 89/06698) nachgewiesen werden.

**Patentansprüche**

1. Verfahren zur spezifischen Vervielfältigung von Nukleinsäure-templates durch Hybridisierung von zwei Nukleinsäure-primern an dieses template, Bildung einer template-komplementären Nukleinsäure mit diesen primern und Transkription der gebildeten Nukleinsäure zu einer einer Mehrzahl von template-analogen Nukleinsäuren, dadurch gekennzeichnet,

   daß das template mit zwei primern, die in gleicher Orientierung angeordnet sind, komplementäre Sequenzen zum template enthalten und von denen der zweite primer an dem dem ersten primer abgewandten Ende eine Transkriptionsinitiationsstelle und eine doppelsträngige Sequenz, an die eine RNA-Polymerase binden kann, trägt, hybridisiert wird,

   die beiden so hybridisierten primer durch Auffüllen der Lücke zwischen ihnen zu einer template-komplementären Nukleinsäure kovalent verknüpft werden

   und Transkripte von dieser template-komplementären Nukleinsäure gebildet werden.

2. Verfahren zum Nachweis von Nukleinsäure-templates durch Hybridisierung von zwei Nukleinsäure-primern an dieses template, Bildung einer template-komplementären Nukleinsäure mit diesen primern und Transkription der gebildeten Nukleinsäure zu einer Mehrzahl von template-analogen Nukleinsäuren, dadurch gekennzeichnet, daß das template mit zwei primern, die in gleicher Orientierung angeordnet sind, komplementäre Sequenzen zum template enthalten und von denen der erste primer an dem zweiten primer abgewandten Ende eine Transkriptionsinitiationsstelle und eine doppelsträngige Sequenz, an die eine RNA-Polymerase binden kann, trägt, hybridisiert wird, die beiden so hybridisierten primer durch Auffüllen der Lücke zwischen ihnen zu einer template-komplementären Nukleinsäure kovalent verknüpft werden, Transkripte von dieser template-komplementären Nukleinsäure gebildet werden und diese Transkripte durch in an sich bekannter Weise nachgewiesen werden.

3. Verfahren nach den Ansprüchen 1 oder 2, dadurch gekennzeichnet, daß die gebildeten Transkripte erneut als templates zur Hybridisierung mit den primern verwendet werden und das Vervielfältigungsverfahren wiederholt wird.

4. Verfahren nach den Ansprüchen 1 bis 3, dadurch gekennzeichnet, daß vor Bildung der Transkripte die Hybridisierung zwischen template und template-komplementärer Nukleinsäure aufgehoben wird.

5. Verfahren nach Anspruch 4, dadurch gekennzeichnet, daß die Hybridisierung durch RNaseH aufgehoben wird.

6. Verfahren nach den Ansprüchen 1 - 5, dadurch gekennzeichnet, daß als template DNA verwendet wird.

7. Verfahren nach den Ansprüchen 1 - 5, dadurch gekennzeichnet, daß als template RNA verwendet wird.

8. Verfahren nach den Ansprüchen 1 - 6, dadurch gekennzeichnet, daß das template vor der Hybridisierung mit den primern in die einzelsträngige Form überführt wird.

9. Verfahren nach den Ansprüchen 1 - 6, dadurch gekennzeichnet, daß ein doppelsträngiges DNA-template mit einer oder mehreren Restriktionsendonukleasen gespalten und in die einzelsträngige Form überführt wird.

10. Verfahren nach den Ansprüchen 1 - 9, dadurch gekennzeichnet, daß die doppelsträngige Sequenz des zweiten primers eine Länge von je 17 - 100 komplementären Basen besitzt.

11. Verfahren nach Anspruch 10, dadurch gekennzeichnet, daß die komplementären Teile des Doppelstrangs über ein Nukleinsäurefragment mit 5 - 100 Basen Länge miteinander verbunden sind.

12. Verfahren nach den Ansprüchen 1 - 11, dadurch gekennzeichnet, daß im zweiten primer das 5'-Ende phosphoryliert ist und das dem ersten primer zugewandte 3'-Ende des Doppelstrangs mit einem Didesoxynucleotid endet.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | FEBS LETTERS, Band 212, Nr. 2, Februar 1987, Seiten 271-275, Elsevier Science Publishers B.V. (Biomedical Division); G. KRUPP et al.: "Simplified in vitro synthesis of mutated RNA molecules. An oligonucleotide promoter determines the initiation site of T7RNA polymerase on ss M 13 phage DNA" <br> * Zusammenfassung; Seite 271, Spalte 2, Zeilen 13-20; Seite 272, Spalte 2, Zeile 15 - Seite 273, Spalte 2, Zeile 14; Figuren 1,2 * <br> — — — | 1,11,12 | C 12 Q 1/68 <br> C 12 N 15/10 |
| Y | IDEM <br> — — — | 2-5 | |
| Y | PROC. NATL. ACAD. SCI. USA, Band 87, März 1990, Seiten 1874-1878; J.C. GUATELLI et al.: "Isothermal, in vitro amplification of nucleic acids by a multienzyme reaction modeled after retroviral replication" <br> * Zusammenfassung; Figur 1 * <br> — — — | 2-5 | |
| A | EP-A-0 303 155   (MERCK PATENT GmbH) <br> * Zusammenfassung; Spalte 2, Zeile 32 - Spalte 4, Zeile 54 * <br> — — — | 1,9 | |
| A | WO-A-9 001 068   (LIFE TECHNOLOGIES INC.) <br> * Seite 6, Zeile 12 - Seite 8, Zeile 24; Seite 20, Zeile 11 - Seite 23, Zeile 11 * <br> — — — | 1,2 | RECHERCHIERTE SACHGEBIETE (Int. Cl.5) <br><br> C 12 Q |
| A | NUCLEIC ACIDS RESEARCH, Band 17, Nr. 2, 25. Januar 1989, Seiten 711-722, Eynsham, Oxford, GB; G.F. JOYCE et al.: "A novel technique for the rapid preparation of mutant RNAs" <br> * Zusammenfassung; Seite 715, Zeilen 7-36; Figur 1 * <br> — — — | 1 | |
| P,Y | EP-A-0 369 775   (LIFECODES CORP.) <br> * Das ganze Dokument; insbesondere Seite 3, Zeilen 10-45; Ansprüche * <br> — — — — — | 2 | |

Der vorliegende Recherchenbericht wurde für alle Patentansprüche erstellt

| Recherchenort | Abschlußdatum der Recherche | Prüfer |
|---|---|---|
| Den Haag | 21 Juni 91 | LUZZATTO E.R.P.G.A. |